# EUROPEAN PATENT APPLICATION

(11) **EP 4 345 772 A1**
(43) Date of publication of application: **03.04.2024**
(21) Application number: 23191135.5
(22) Date of filing: 11.08.2023
(51) Int. Cl.: G06V 20/40, G06V 20/52, G06V 40/10

(54) **A COMPUTER IMPLEMENTED METHOD AND SYSTEM FOR IDENTIFYING AN EVENT IN VIDEO SURVEILLANCE DATA**

(30) Priority: 29.09.2022 GB 202214296
(71) Applicant: Milestone Systems A/S, 2605 Brøndby (DK)
(72) Inventor: LI, Zenjie, 2605 Brøndby (DK); NASROLLAHI, Kamal, 2605 Brøndby (DK)
(74) Representative: Canon Europe Limited

(57) **Abstract**

A computer implemented method for identifying an event by processing video surveillance data from a video camera having a field of view uses a trained machine learning algorithm to detect a person in the video surveillance data and determine the location of the person in the field of view. A trained machine learning algorithm is used to classify a posture of the detected person and a detection zone within the field of view around the location of the detected person is defined. A trained machine learning algorithm is used to search for an object of a predetermined type overlapping the detection zone. An event is identified based on the posture detection and the result of the object detection.

## Description

### Technical field of the Invention

The present invention relates to a method, system and computer program for identifying anomalous events in video surveillance data, using machine learning algorithms.

### Background of the Invention

Many video analytics software modules that utilise machine learning algorithms are available which can analyse video surveillance data and detect specific objects or activity. These software modules can be provided in a video management system that processes data from multiple cameras, but as processing capacity in cameras increases, they are increasingly provided in the cameras themselves ("on edge"). Analytics modules on the edge identify objects or activity in the video data from the camera, and generate metadata describing the detected objects or activity and indicating a time and position in the frame (eg bounding box coordinates) where the objects or activity have been detected. The metadata is sent to the video management system where it is stored on a recording server with the video data. Metadata can be used by a client device to generate alerts, provide visual indications on live or recorded video or can be used to search stored video data.

An example of object detection would be a human detection algorithm, which can identify humans in the video data and also particular characteristics of the identified humans such as gender, age, colour of clothing or particular clothing items (eg wearing a hat). There are also human detection algorithms that can detect humans and classify their posture or activity as, for example, sitting, standing, lying, running etc..

Other video analytics software modules can detect and classify objects such as furniture in a room which can be classified as, for example, bed, chair, lamp, table etc..

Other video analytics software modules can detect and identify activities or behaviour. An example would be a video analytics module used to analyse video data from a shopping mall which can identify suspicious behaviour such as loitering, shoplifting or pickpocketing. These can be more complicated to detect as they may involve interactions between multiple humans or between humans and objects.

One example of a useful application of video analytics would be in a hospital or care home environment where it would be useful to provide a video analytics module that can identify a patient in distress, for example someone falling over. However, this is a more complex situation to identify because in a hospital there may be a mixture of people standing, sitting and lying and it is not straightforward to identify someone who has fallen over compared to someone who is lying in a bed.

US7,612,666 proposes a solution in which the field of view of a camera is divided into a plurality of zones, each having an algorithm corresponding to movement in that zone. Therefore, a bed zone can be identified, and certain behaviour in that zone can trigger an alarm, such as movement which could indicate someone falling from the bed. However, one problem with this approach, particularly in a hospital environment, is that furniture may often be moved, patients may be lying on gurneys, and therefore the zones may not be fixed.

### Summary of the Invention

The present invention provides a computer implemented method according to claim 1.

Preferred features of the method are set out in claims 2 to 8.

The present invention also provides a system for analysing video surveillance data according to claim 9.

The present invention also provides a video management system according to claim 12.

Therefore, the present invention starts with the detection of a person, and determines an event based on a relationship between a classified posture of the person and a detected object in the vicinity of the person. Thus the invention does not require any prior knowledge of positioning of objects or definition of zones. Detection algorithms for humans are generally well developed and accurate and therefore it is preferable to start with the detection of a human and, only when a human of the predetermined posture has been detected, then look for objects in the vicinity of the human when the search area has been narrowed down, rather than start with the detection of objects. The search area for the object detection is defined based on the detection zone.

In the case of fall detection, the objects being detected would be beds or similar furniture and machine learning algorithms for detection of such objects are less accurate due to the large diversity of types of furniture and placing orientations.

A video management system may receive video surveillance data in which persons and postures have already been identified by a machine learning algorithm in the camera, and a description of the persons and postures and their location in the video have been included in metadata. Thus, the video management system can search the metadata for a person having a predetermined posture, determine the location of the person, define a detection zone and use a trained machine learning algorithm to search the video data for an object overlapping the detection zone.

### Brief Description of the Drawings

Embodiments of the present invention will now be described, by way of example only, with reference to the accompanying drawings in which:
Figure 1 shows a video surveillance system in which the present invention can be implemented;
Figures 2A and 2B are video surveillance images including a person who has had a fall;
Figure 2C is a video surveillance image including a person lying in a bed;
Figure 4 is a flow chart of a method according to a first embodiment of the present invention; and
Figure 5 is a flow chart of a method according to a second embodiment of the present invention.

### Detailed Description of the Invention

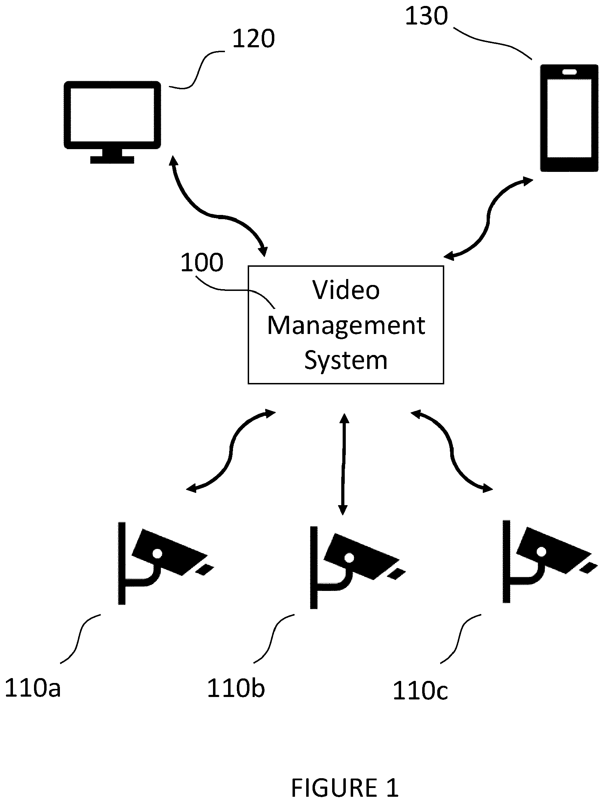

Figure 1 shows an example of a video surveillance system in which embodiments of the invention can be implemented.

The system comprises a video management system (VMS) 100, a plurality of video surveillance cameras 110a, 110b, 110c and at least one operator client 120 and/or a mobile client 130.

The VMS 100 may include various servers such as a management server, a recording server, an analytics server and a mobile server. Further servers may also be included in the VMS, such as further recording servers or archive servers. The VMS 100 may be an "on premises" system or a cloud-based system or a hybrid system.

The plurality of video surveillance cameras 110a, 110b, 110c send video data as a plurality of video data streams to the VMS 100 where it may be stored on a recording server (or multiple recording servers). The operator client 120 is a terminal which provides an interface via which an operator can view video data live from the cameras 110a, 110b, 110c, or recorded video data from a recording server of the VMS 100.

The VMS 100 can run analytics software for image analysis, for example software including machine learning algorithms for object or activity detection. The analytics software may generate metadata which is associated with the video data and which describes objects and/or activities which are identified in the video data.

Video analytics software modules may also run on processors in the cameras 110a, 110b, 110c. In particular, a camera may include a processor running a video analytics module including a machine learning algorithm for identification of objects or activities. The video analytics module generates metadata which is associated with the video data stream and defines where in a frame an object or activity has been detected, which may be in the form of coordinates defining a bounding box, and which will also include a time stamp indicating the time in the video stream where the object or activity has been detected. The metadata may also define what type of object or activity has been detected eg person, car, dog, bicycle, and/or characteristics of the object (eg colour, speed of movement etc). The metadata is sent to the VMS 100 and stored and may be transferred to the operator client 120 or mobile client 130 with or without its associated video data. A search facility of the operator client 120 or mobile client 130 allows a user to look for a specific object, activity or combination of objects and/or activities by searching the metadata. Metadata can also be used to provide alerts to an operator to alert the operator of objects or activities in the video while the operator is viewing video in real time. Metadata generated in the camera can be used by further analytics modules in the VMS 100 or the operator client 120 or mobile client 130. For example, metadata generated by object recognition modules in the cameras can be used in the VMS 100 or the operator client 120 or mobile client 130 to identify events based on the relationships between objects identified by the analytics modules in the cameras.

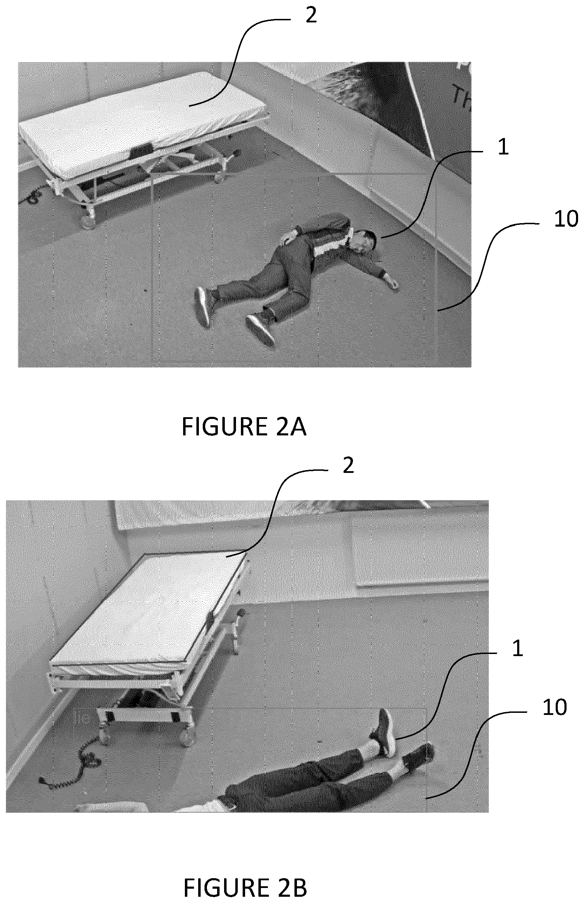

Figures 2A and 2B show images from a video surveillance camera which illustrate the type of events which may be detected in an embodiment of the present invention. In both Figures 2A and 2B, a person 1 has fallen and is lying on the floor in a room including a bed 2.

In this embodiment of the invention, first the person 1 is detected and identified in the image using a trained machine learning algorithm, and the posture of the person 1 is classified. In this case, the person is classified as being in a lying posture.

It is known that person detection algorithms are mature and reliable due to the abundance of training data, relative homogeneity of humans as objects and plenty of studies focusing on human features. Typical known algorithms include region-based detections, such as faster R-CNN, and single-shot methods, such as the YOLO series. Due to the excellent balance between YOLO and its variants' accuracy and speed, they are widely used in video analytics today.

For each detected person, single image posture classification can be achieved with a Convolutional Neural Network with the image as the input and a number for each class as the output. For example, in the case of two posture classes, lying and non-lying, the output result might be 0.8 for lying while 1.2 for non-lying. Since 1.2 is greater than 0.8, the posture will be classified as "non-lying". An example of such a classifier is ResNet and its variants. These models first extract features from an image (in this case from the image sub-region encompassing the detected person) based on Convolutional Neural Networks. Then one or more fully connected layers are added to classify into the target classes (in this case "lying" and "not-lying"). Such classifiers can be readily extended for an image series by inputting multiple images altogether and applying extra layers immediately after the input layer in the network architecture.

A person's posture can also be classified in a more explicit way, which is advantageous in case a person is partially occluded by other objects. After the bounding box of a person is detected, the body keypoints, such shoulders, elbows, wrists, hips, knees, and ankles, can be detected by a body keypoint detector, such as the keypoint R-CNN model implemented in the Detectron2 library. The pre-trained keypoint detection models need fine-tuning, though, so they will work for persons with lying, crouching, falling or similar postures. Then a multilayer perceptron network can be constructed for the posture classification. The input will be the detected body keypoints, while the output will be the posture classes. Like feature extraction networks described above, such classifiers can be readily extended for an image series by e.g., concatenating the detected keypoints for those image series as in the classification input.

As part of the person detection, a detection zone 10 is defined around the detected person 1. This can be in the form of a bounding box, and the machine learning algorithm can generate metadata defining the bounding box eg by coordinates of two diagonally opposed corners in the frame. The metadata also includes data identifying the object (eg person) and may include characteristics of the object (eg posture, gender, colour of clothing).

In this embodiment, if a person 1 having a posture classified as lying is identified as present in the image, a trained machine learning algorithm is then used to identify objects in the vicinity of the person that overlap the detection zone 10, particularly furniture for resting. Furniture for resting includes beds, gurneys or any other item of furniture that a person could lie on, including a sofa or chaise longue. In the examples in Figures 2A and 2B, a gurney or hospital bed 2 is present in the room. It is determined whether any detected bed overlaps the detection zone 10, and the extent of the overlap and the orientation of the bed 2 and the person 1 can be used to determine whether the person 1 is lying on the bed 2. For example, when a bed is detected, at least two diagonal corners are determined. For better reliability, all the four corners or two diagonal corners plus the rotation angle can be detected. This determines a bounding box for the bed. Then, the overlap with the bounding box defining the person's position is determined. Therefore, the bounding box for the person is set to be the detection zone. If a significant portion of the person's bounding box overlaps with the bed's bounding box by more than a predetermined amount, such as >50%, it is determined that the person is on the bed.

To enhance the reliability of the aforementioned overlapping test, a segmentation algorithm like mask RCNN can be employed to detect resting furniture. So, the furniture will be defined at the pixel level rather than being limited to two diagonal corners for a bounding box. The overlapping percentage will be calculated in a similar way. Segmentation is a heavier process than object detection. Since resting furniture does not move as frequently as a person, there is no need to segment it every time a person is detected. For instance, one can detect a person at a frame rate of 10 FPS while only segmenting the resting furniture at a frame rate of 1 FPS.

If not, then the person 1 may be lying on the floor and it is determined that a fall event has occurred. If a fall event is determined, then metadata is associated with the video data which indicates a fall event at the position of the person 1. The metadata defines where in the frame the fall event has been detected, which may be in the form of coordinates defining a bounding box. The same bounding box as the bounding box indicating the person can be used. The metadata indicating the fall event may be associated with the same bounding box data that identifies the detected person 1. The metadata also includes a time stamp indicating the time in the video stream. This metadata may then be used to trigger an alarm or an alert, and can be used to search for fall events in recorded video surveillance data.

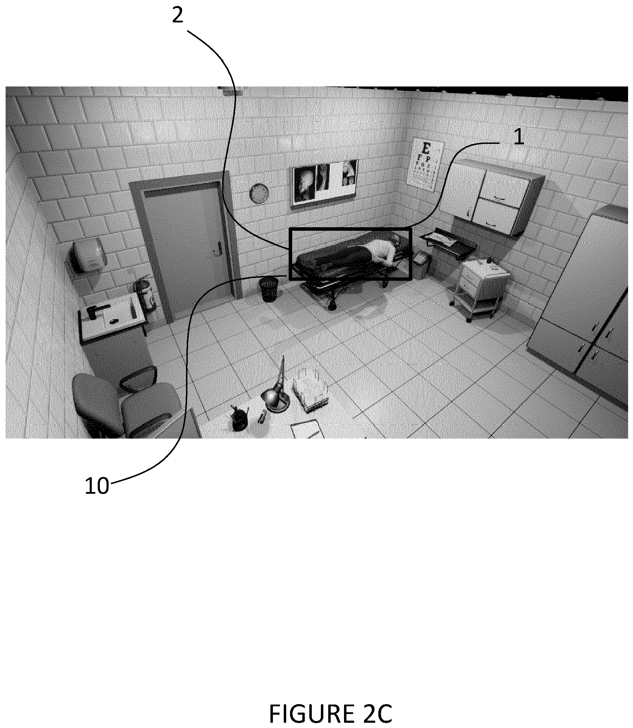

Figure 2C shows an example of an image in which a fall is not determined to have occurred. A person 1 is detected and classified as having a lying posture, and a detection zone 10 is drawn around the person 1. However, a bed 2 is detected which substantially overlaps the detection zone 10 and therefore no fall event is detected.

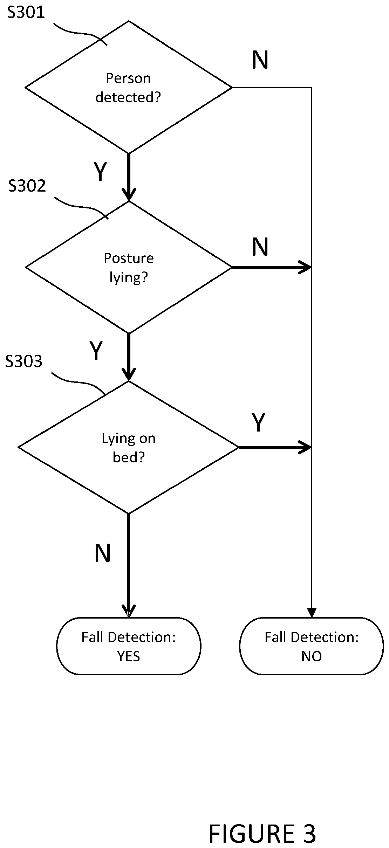

Figure 3 is a flow chart illustrating a method in accordance with one embodiment of the invention used for fall detection.

First, in step S301, the machine learning algorithm is applied to the video surveillance data and it is determined if a person is detected. If a person is detected then, in step S302, the posture of the person is classified. If the posture is not lying, then no fall is detected. If the posture is lying, then in step S303, it is determined if a detection zone defining where the person is detected overlaps with a predetermined object, in this case, an item of furniture for resting (can be a bed, gurney, couch/sofa etc) and the extent of overlap and orientation may be considered to determine if the person is lying on the item of furniture. For example, when a bed is detected, at least two diagonal corners are determined. For better reliability, all the four corners or two diagonal corners plus the rotation angle can be detected. This determines a bounding box for the bed. Then, the overlap with the bounding box defining the person's position is determined. Therefore, the bounding box for the person is set to be the detection zone. If a significant portion of the person's bounding box overlaps with the bed's bounding box, say >50%, it is determined that the person is on the bed.

If it is determined that the person is not lying on the item of furniture (or there is no item of furniture), then a fall event is detected.

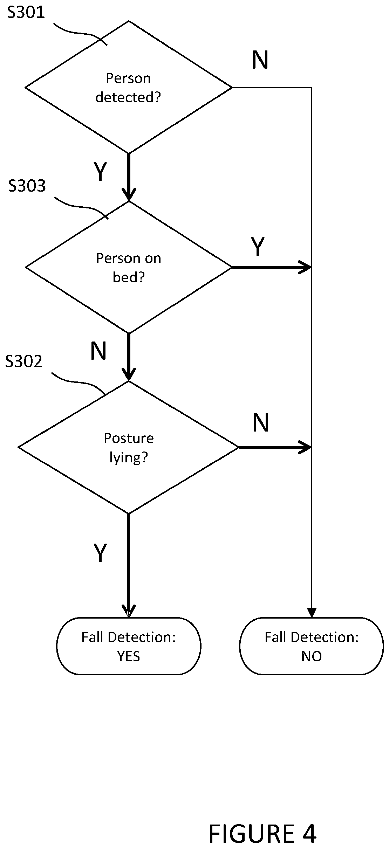

Figure 4 is a flow chart illustrating an alternative method in accordance with another embodiment, which is similar to the embodiment of Figure 3, but with steps S302 and S303 reversed.

As in the embodiment of Figure 3, first, in step S301, the machine learning algorithm is applied to the video surveillance data and it is determined if a person is detected. If a person is detected then, in step S303, it is determined if a detection zone defining where the person is detected overlaps with a predetermined object, in this case, an item of furniture for resting (this can be a bed, gurney, couch/sofa etc). If there is an item of furniture for resting then no fall is detected. The extent of the overlap may be considered in this determination. If an item of furniture for resting is not detected, then in step S302, the posture of the person is classified. If the posture is not lying, then no fall is detected. If the posture is lying, then it is determined that a fall event is detected.

Although the above embodiments describe separate steps, some of the steps may be carried out together by the same machine learning algorithm. For example, in the embodiment of Figure 3, steps S302 and S303 can be carried out in a single step where a classifier directly decides if a person is lying in an unsafe zone (not on a bed) or a safe zone (on a bed).

The above description illustrates examples of methods in accordance with the invention. There are various ways in which methods can be implemented in a system such as that of Figure 1.

In one example, all of the steps of Figures 3 or 4 can be carried out by video analytics modules in a processor in the camera 110a, 110b, 110c itself. In this case, the person detection, posture detection, furniture detection and event detection are all carried out in the camera, and the video data is streamed to the VMS 100 with metadata that identifies detected events. The metadata identifying an event will identify the type of event, the location within the field of view and a time stamp. Other metadata may also be included that relates to component parts of the method, such as bounding boxes indicating detected persons or objects with descriptions of classifications (eg posture classifications like sitting or lying or object classifications). Metadata indicating an event (eg a fall) can be used by the VMS 100 to trigger an alarm or alert to an operator viewing video data via an operator client 120 or mobile client 130, or may be stored and used later to search for events by an operator searching video data via the operator client 120 or mobile client 130.

In another example, the video cameras 110a, 110b, 110c may simply stream the video data to the VMS 100, and all of the analytics steps may be carried out by a processor in the VMS 100 which can generate and store all of the same metadata discussed above, generate alerts, or carry out metadata searches on stored video data.

It is also possible that some of the steps may be carried out in a processor in the camera 110a, 110b, 110c itself, and some steps in a processor in the VMS 100. For example, the camera 110a, 110b, 110c could include person detection which detects humans and classifies posture, and generates metadata including bounding boxes defining the person location and metadata identifying the classified posture, and sends this metadata with the video data to the VMS 100. In this example, therefore, steps S301 and S302 of Figure 3 are carried out in the camera. A further analytics module running on a processor in the VMS 100 could then use the metadata generated in the camera to identify persons having a lying posture, and carry out step S303, and make a final event detection.

Although the present invention has been described in the context of fall detection, there are other events that could be detected starting from detection of a person and taking into account the posture of the detected person and their context in relationship to an object. For example, in video monitoring of a car park, a combination of a person bending over in the vicinity of a car could indicate tampering.

While the present invention has been described with reference to embodiments, it is to be understood that the invention is not limited to the disclosed embodiments. The present invention can be implemented in various forms without departing from the principal features of the present invention as defined by the claims.

## Claims

1. A computer implemented method for identifying an event using video surveillance data from a video camera (110a, 110b, 110c) having a field of view comprising the steps of:
(1) using a trained machine learning algorithm to detect a person (1) in the video surveillance data and determine a location of the person in the field of view;
(2) using a trained machine learning algorithm to classify a posture of the detected person, and, only when the posture is classified as a predetermined posture, carrying out the further steps of:
(3) defining a detection zone (10) within the field of view around the location of the detected person;
(4) using a trained machine learning algorithm to search for an object (2) of a predetermined type overlapping the detection zone (10); and
(5) identifying an event based on the posture detection and the result of the object detection.

2. The method according to claim 1, further comprising a step of generating metadata identifying the event, a time of the event in the video, and a location of the event within the field of view.

3. The method according to claim 1 or 2, wherein the search in step (4) is limited to an area defined by the detection zone (10).

4. The method according to any one of the preceding claims, wherein, in step (5), when the posture is classified as a predetermined posture and no object of the predetermined type is detected overlapping the detection zone, then it is determined that a specific event has occurred.

5. The method according to claim 4, wherein, if an object of the predetermined type is detected overlapping the detection zone by less than a predetermined amount, then it is determined that the specific event has occurred.

6. The method according to claim 4 or 5, wherein the predetermined posture is a lying posture and the object is an item of furniture for resting.

7. The method according to any one of the preceding claims, wherein steps (2) to (4) are carried out by the same trained machine learning algorithm.

8. A computer program comprising code which, when run on a processor causes it to carry out the method according to any one of claims 1 to 7.

9. A system for processing video surveillance data from a video camera having a field of view, the system comprising at least one processing unit configured to receive and process video surveillance data to:
(1) use a trained machine learning algorithm to detect a person in the video surveillance data and determine a location of the person in the field of view;
(2) use a trained machine learning algorithm to classify a posture of the detected person, and, only when the posture is classified as a predetermined posture, to:
(3) define a detection zone within the field of view around the location of the detected person;
(4) use a trained machine learning algorithm to search for an object of a predetermined type overlapping the detection zone; and
(5) identify an event based on the posture detection and the result of the object detection.

10. The system according to claim 9, comprising the video camera, wherein the at least one processing unit includes a processing unit in the camera, wherein at least steps (1) and (2) are carried out by the processing unit in the camera.

11. The system according to claim 9 or 10, comprising a video management system including at least one server configured to receive video data from the camera and wherein the at least one processing unit includes a processing unit in the video management system, wherein at least steps (4) and (5) are carried out by the processing unit in the video management system.

12. A video management system (100) for processing video surveillance data from a video camera having a field of view, the video management system comprising a processing unit configured to:
(1) receive the video surveillance data including metadata relating to detected objects in the video data;
(2) using the metadata to identify a person (1) having a predetermined posture and determine a location of the person in the field of view;
(3) define a detection zone (10) within the field of view around the location of the detected person;
(4) use a trained machine learning algorithm to search for an object of a predetermined type overlapping the detection zone; and
(5) identify an event based on the result of the object detection.

13. The video management system according to claim 12, wherein the search in (4) is limited to an area defined by the detection zone.
